# EUROPEAN PATENT SPECIFICATION

(11) **EP 1 481 991 B1**
(45) Date of publication and mention of the grant of the patent: **17.01.2007**
(21) Application number: 04251109.7
(22) Date of filing: 27.02.2004
(51) Int. Cl.: C07K 14/715, A61K 39/35, C12N 15/29, G01N 33/53

(54) **Allergenic protein of natural rubber**
Allergenes Kautschuk-Protein
Protéine allèrgenique de caoutchouc

(30) Priority: 28.02.2003 MY 0300734
(43) Date of publication of application: 01.12.2004
(73) Proprietor: Malaysian Rubber Board, Kuala Lumpur 50450 (MY)
(72) Inventor: Mad Arif, Siti Arija Malaysian Rubber Board, Sungai Buloh Selangor 47000 (MY); Chew, Nyu Ping Malaysian Rubber Board, Sungai Buloh Selangor 47000 (MY); Yeang, Hoong Yeet Malaysian Rubber Board, Sungai Buloh Selangor 47000 (MY)
(74) Representative: Lord, Hilton David

(56) References cited:
- EP-A- 0 704 457
- WO-A-01/23429
- WO-A-01/61305
- WO-A-95/07298
- YEANG H Y ET AL: "Allergenic proteins of natural rubber latex." METHODS (SAN DIEGO, CALIF.) MAY 2002, vol. 27, no. 1, May 2002 (2002-05), pages 32-45, XP002295041 ISSN: 1046-2023
- ALENIUS H ET AL: "PROHEVEIN FROM THE RUBBER TREE (HEVEA BRASILIENSIS) IS A MAJOR LATEX ALLERGEN" CLINICAL AND EXPERIMENTAL ALLERGY, BLACKWELL SCIENTIFIC PUBLICATIONS, LONDON, GB, vol. 24, 1995, pages 659-665, XP009011560 ISSN: 0954-7894
- SUNDERASAN E ET AL: "LATEX B-SERUM BETA-1,3+-GLUCANASE (HEV B II) AND A COMPONENT OF THE MICROHELIX (HEV B IV) ARE MAJOR LATEX ALLERGENS" JOURNAL OF NATURAL RUBBER RESEARCH, KUALA LUMPUR, MY, vol. 10, no. 2, 1995, pages 82-99, XP009011506
- BEEZHOLD D H ET AL: "MUTATIONAL ANALYSIS OF THE IGE EPITOPES IN THE LATEX ALLERGEN HEV B 5" JOURNAL OF ALLERGY AND CLINICAL IMMUNOLOGY, MOSBY - YEARLY BOOK, INC, US, vol. 107, no. 6, June 2001 (2001-06), pages 1069-1076, XP008026033 ISSN: 0091-6749
- ARIF SITI ARIJA M ET AL: "Isolation and characterization of the early nodule-specific protein homologue (Hev b 13), an allergenic lipolytic esterase from Hevea brasiliensis latex." THE JOURNAL OF BIOLOGICAL CHEMISTRY. 4 JUN 2004, vol. 279, no. 23, 4 June 2004 (2004-06-04), pages 23933-23941, XP002295042 ISSN: 0021-9258

## Description

### 1. Technical Field of The Invention

The present invention relates in general to a protein.

### 2. Background of The invention

By the late 1980s and into the 90s, reports began to be received with increasing frequency in Europe and America of allergic reactions occurring among users of surgical and examination gloves made of latex and among spina bifida patients. The significant increase in the number of reports of latex allergy in the last decade has also been attributed to increased usage of latex gloves in healthcare in tandem with the rising cases of AIDS. Sensitisation to latex among healthcare workers is clearly work-related, the main cause being latex gloves, or specifically, the allergenic protein in latex gloves. Nevertheless, numerous cross-sensitivities between latex protein allergens and various food and pollen allergens are known. It is therefore not improbable that many latex-allergic patients may have been initially sensitised not only by proteins from latex products, but also by proteins from other sources.

There are hundreds of proteins found in natural rubber latex. Of these, only a small handful is allergenic (able to induce allergy). There has been much interest in identifying the proteins in Hevea latex responsible for latex allergy and considerable effort is expended on isolating and purifying the allergenic proteins from Hevea latex or latex products. Other than from the academic standpoint, elucidation of the major allergens in latex would enable antibodies to be developed against these proteins. Availability of the antibodies would facilitate the development of latex immunoassays, both for laboratory and commercial use. There are two main types of latex immunoassays.

### 1. Immunoassays for latex allergy diagnosis

These diagnostics are used to determine if someone is allergic or sensitized to latex. The assays can either be of the *in vitro* format (usually a serological test) or of the *in vivo* format (skin prick tests). These assays are used in research and in healthcare.

### 2. Immunoassays for the quantitation of latex allergens in manufactured products

These quantitative assays determine the amount of allergenic proteins present in latex products. They are used for testing latex products such as latex gloves to determine the content of extractable latex allergens. Such immunoassays would be very valuable in latex product manufacture, particularly in the aspects of standardisation and quality control and quality assurance. The prospective customers for such immunoassays would be latex product manufacturers and regulatory agencies charged with the responsibility of ensuring product specification compliance.

Identification of the major latex allergens serves another useful function in healthcare. Purified latex allergens can be used in immunotherapy to de-sensitise latex allergic patients. When successfully undertaken, the patient no longer develops an allergic reaction to latex. This is especially important where the patient works in an environment (e.g. in healthcare) where latex products are ubiquitous.

Today, the International Union of Immunological Societies (IUIS) recognises ten latex allergens, Hev b 1 to Hev b 10. (There are other latex proteins under consideration by the IUIS.) Although there is effort being made to look for significant latex protein allergens, many researchers believe that most of the major latex protein allergens have been accounted for.

In 1995, Dr Donald Beezhold in his paper presented at *Int Conf on Latex Protein Allergy: The Latest Position* announced a new latex allergen that had partial protein homology to patatin, the major storage protein of potatoes. This 43 kDa protein is later assigned the WHO/IUIS name Hev b 7. When a recombinant version of Hev b 7 became available, it is found to be reactive with IgE from latex allergic patients. However, the proportion of patients that are sensitised to recombinant Hev b 7 is much lower than expected. Western blots that showed an active protein band around 43 kDa protein is much more commonly encountered than could be explained by IgE binding to Hev b 7. Hence, the recombinant Hev b 7 could not account for the very frequent occurrence of latex sensitivity to a protein of about 43 kDa. It is, therefore, possible that another unknown latex allergen of around 43 kDa existed. The search for this new and unknown protein has culminated in the present invention. This protein is allergenic in nature in that contact with allergenic latex protein (ALP) can induce an allergic reaction in persons sensitized to this protein

### 3. Summary of the Invention

The present invention relates to a protein originating from latex that can induce an allergic reaction in persons sensitized to the protein.

Thus, in a first aspect, the present invention provides an allergenic latex protein (ALP), characterised in that the protein has at least 80% sequence identity with SEQ ID NO. 12.

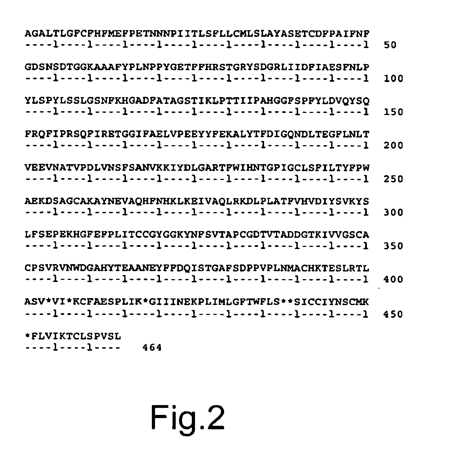

Preferably, the protein or its molecular variant characterized in that the protein has the following properties:
a) has a molecular weight of about 42,000 Dalton;
b) has an isoelectric point of about 4.7;
c) binds with IgE of patients sensitized to the protein; and
d) contains the amino acid sequence as in Figure 2 (SEQ ID NO. 12) or minor variations of these amino acid sequence that do not result in the allergenic properties of the protein being substantially altered.

The second aspect of the present invention provides for a process for obtaining a protein or its molecular variant where the process comprises the following steps :
a) centrifuging the latex for obtaining the bottom fraction;
b) freeze-thawing the bottom fraction for obtaining the latex B-serum; and
c) isolating and purifying the protein from the B-serum obtained in (b).

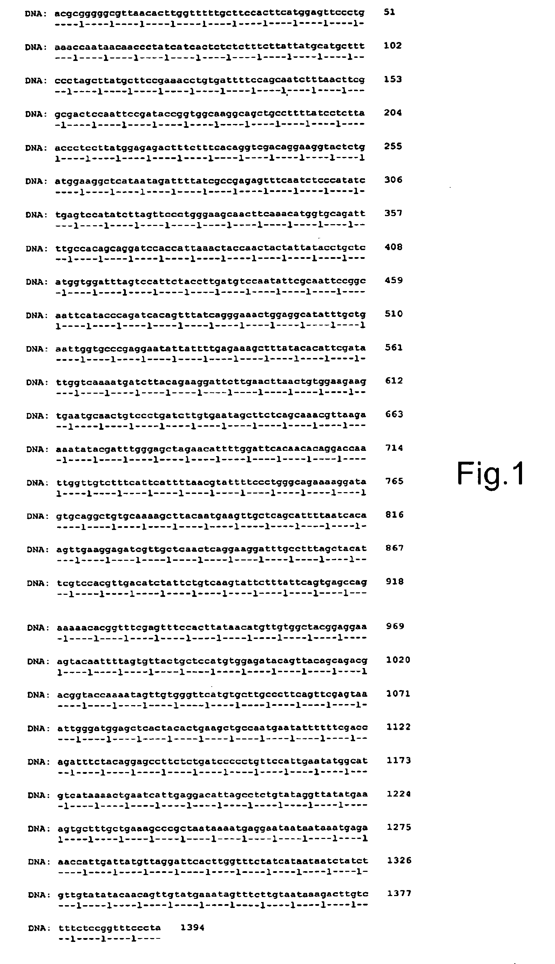

Further, the present invention provides for a DNA sequence encoding the protein or a portion of the protein where the DNA sequence is as Figure 1 or minor variations of this sequences.

Also, the present invention provides a method for the production of a protein or its molecular variants in recombinant form by inserting the DNA encoding the protein or a variant of the protein into an appropriate vector and inducing the vector to express recombinant protein or in recombinant form of the said variant of the protein, whereby in this case, the amino acid sequence of the above translated DNA sequence are as in Figure 2 (SEQ ID NO. 12).

### 4. Brief Description of the Figures

Figure 1 shows the DNA sequence of the full length cDNA clone encoding the ALP. Figure 1 is also shown in SEQ ID NO. 11.
Figure 2 shows the amino acid sequence of the ALP derived from the translation of the cDNA clone encoding the ALP. Figure 2 is also shown in SEQ ID NO. 12.
Figure 3 shows the matrix assisted laser desorption ionisation mass spectrometry (MALDI-MS) spectrum of the allergenic latex protein, ALP.
Figure 4 shows a Western blot of ALP after separation of the protein by SDS-polyacrylamide gel electrophoresis. The protein is stained with Coomassie Blue to show the presence and electrophoretic migration of ALP (lane 2). Binding of human IgE of patients sensitised to ALP on the Western blot (lane 3), polyclonal antibodies developed against ALP (lane 4), and a monoclonal antibody developed against ALP (lane 5).
Figure 5 shows a Western blot of ALP after separation of the protein by SDS-polyacrylamide gel electrophoresis. The protein is stained with Coomassie Blue to show the presence and electrophoretic migration of ALP (lane 2). A reaction specific for the presence of carbohydrates is carried out to demonstrate glycosylation of ALP (lane 3).
Figure 6 shows a Western blot of the recombinant MBP-ALP fusion protein after separation by SDS-PAGE. The protein is stained with Coomassie Blue to show the electrophoretic migration of the recombinant ALP fusion protein (lane 3). Binding of recombinant ALP to monoclonal (Lane 4) and polyclonal antibodies (lane 5) developed against native ALP is also shown.

### Detailed description of the invention

The present invention relates to a protein isolated from the B-serum of centrifuged latex obtained by tapping the rubber tree, *Hevea brasiliensis.* The following description details how the protein can be isolated, purified and characterized, and how it might be used in cloning the cDNA encoding the protein, how recombinant versions of the protein can be obtained, how antibodies might be developed from the protein and how the protein can be used in immunoassay and immunotherapy.

The invention extends to any peptide or polypeptide, which terms are used interchangeably herein, except where otherwise required, which has substantial identity with precursor or mature ALP. Polypeptides with substantial identity include variants, such as allelic variants.

A "variant" as the term is used herein, may refer to a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

Within the variants we include altered polypeptides and polynucleotides. "Altered" nucleic acid sequences encoding ALP include those sequences with deletions, insertions, or substitutions of different nucleotides, resulting in a polynucleotide the same as ALP or a polypeptide with at least one functional characteristic of ALP. Included within this definition are polymorphisms which may or may not be readily detectable using a particular oligonucleotide probe of ALP, and improper or unexpected hybridisation to allelic variants, with a locus other than the normal chromosomal locus for the polynucleotide sequence encoding ALP. The encoded protein may also be "altered," and may contain deletions, insertions, or substitutions of amino acid residues which produce a silent change and result in a functionally equivalent ALP. Deliberate amino acid substitutions may be made on the basis of similarity in polarity, charge, solubility, hydrophobicity, hydrophilicity, and/or the amphipathic nature of the residues, as long as the biological or immunological activity of ALP is retained. For example, negatively charged amino acids may include aspartic acid and glutamic acid, positively charged amino acids may include lysine and arginine, and amino acids with uncharged polar head groups having similar hydrophilicity values may include leucine, isoleucine, and valine; glycine and alanine; asparagine and glutamine; serine and threonine; and phenylalanine and tyrosine.

Fragments of ALP may take various forms, and are also disclosed in the description. A fragment is a polypeptide having an amino acid sequence that entirely is the same as part, but not all, of the amino acid sequence of ALP, or a variant thereof. Fragments may be "free-standing", or comprised within a larger polypeptide of which they form a part or region, most preferably as a single continuous region. Representative examples of polypeptide fragments include, for example, fragments from about amino acid number 1-20, 21-40, 41-60, 61-80, 81-100, and 101 to the end of ALP polypeptide. In this context "about" includes the particularly recited ranges larger or smaller by several, 5, 4, 3, 2 or 1 amino acid at either extreme or at both extremes.

Preferred fragments include, for example, truncation polypeptides having the amino acid sequence of the ALP polypeptide, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterised by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding regions, and high antigenic index regions. Other preferred fragments are biologically active fragments. Biologically active fragments are those that mediate receptor activity, including those with a similar activity or an improved activity, or with a decreased undesirable activity. Also included are those that are antigenic or immunogenic in an animal, especially in a human if it is desired to block ALP binding, for example.

Preferably, all of these polypeptide fragments retain the biological activity of ALP, including antigenic activity. Variants of the defined sequence and fragments also form part of the present description. Preferred variants are those that vary from the referents by conservative amino acid substitutions - *i.e.*, those that substitute a residue with another of like characteristics. Typical such substitutions are among Ala, \/ at, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, or 1-2 amino acids are substituted, deleted, or added in any combination.

Substantially identical polypeptides form a preferred embodiment of the present invention, and include those with variation in at least one part of the molecule, with the option of retaining unchanged another part of the molecule. Thus, for example, if the substantially identical polypeptide is to mimic the activity of ALP, then it may be preferable to retain unchanged particular subregions, while adopting changes in one or more of the other subregions, if desired. The changes may include deletion of such subregions, thereby giving active fragments.

The polypeptides with substantial identity preferably have at least 80% identity with a corresponding polypeptide which is precursor or mature ALP. More preferably there is at least 90% identity, such as at least 95% or at least 98% identity. Sequence identity is suitably determined by a computer programme, though other methods are available. We prefer that identity is assessed using the Gap program from Genetic Computer Group (1994), (see Program Manual for the Wisconsin Package, Version 8, Madison Wisconsin, USA).

"Identity" is a measure of the identity of nucleotide sequences or amino acid sequences. In general, the sequences are aligned so that the highest order match is obtained. "Identity" *per se* has an art-recognised meaning and can be calculated using published techniques. See: *e.g.*: (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed.: Academic Press, Now York, 1993; COMPUTER ANALYSIS OF SEQUENCE DATA, PART 1, Griffin, A.M., and Griffin, H.G., eds., Humana Press, New Jersey, 1994; SEQUENCE ANALYSIS IN MOLECULAR BIOLOGY, von Heinje, G., Academic Press, 1987; and SEQUENCE ANALYSIS PRIMER, Gribskov, M. and Devereux, J., eds., M Stockton Press, New York, 1991).

While there exist a number of methods to measure identity between two polynucleotide or polypeptide sequences, the term "identity" is well known to skilled artisans (Carillo, H., and Lipton, D., *SIAM* J Applied Math (1988) 48:1073). Methods commonly employed to determine identity or similarity between two sequences include, but are not limited to, those disclosed in Guide to Huge Computers, Marlin J. Bishop, ed., Academic Press, San Diego, 1 994, and Carillo, H., and Lipton, D., *SIAM* J Applied Math (1988) 48:1073. Methods to determine identity and similarity are codified in computer programs. Preferred computer program methods to determine identity and similarity between two sequences include, but are not limited to, GCS program package (Devereux, J., *et al.*, Nucleic Acids Research (1984) 12(1):387); BLASTP, BLASTN, FASTA (Atschul, S.F. *et al.,* J Mol. Biol. (1990) 215:403).

As an illustration, by a polynucleotide having a nucleotide sequence having at least, for example, 95% "identity" to a reference nucleotide sequence of SEQ ID NO: 11 is intended that the nucleotide sequence of the polynucleotide is identical to the reference sequence except that the polynucleotide sequence may include up to five point mutations per each 100 nucleotides of the reference nucleotide sequence of SEQ ID NO: 11. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5 or 3 terminal positions of the reference nucleotide sequence or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of SEQ ID NO: 12 is intended that the amino acid sequence of the polypeptide is identical to the reference sequence except that the polypeptide sequence may include up to five amino acid alterations per each 100 amino acids of the reference amino acid of SEQ ID NO: 12. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

The polypeptides of this invention may be embedded within a longer sequence, in the form of a fusion protein, for example. Thus, the polypeptides may be in the form of the "mature" protein, or may be part of a larger protein, such as a fusion protein. It is often advantageous to include an additional amino acid sequence which contains secretory or leader sequences, pro-sequences, sequences which aid in purification, such as multiple histidine residues, or an additional sequence for stability during recombinant production.

The polypeptides which are fragments preferably retain the desired function of ALP. The fragments suitably comprise at least 10 amino acids, more preferably at least 20 amino acids, and typically at least 40 amino acids.

### Example 1. Protein isolation and purification

Fresh latex from *Hevea brasiliensis* trees (clone RRIM 600) is collected into chilled containers. The latex is centrifuged at 19,000 r.p.m. (43,000 g) on a Sorvall RC 5C high-speed centrifuge for 1 h at 4-7°C to separate it into three main fractions: the top fraction which is the rubber cream, the heavy bottom fraction and the C-serum located in between. Latex B-serum is prepared based on the method of Hsia (1958) *Trans Instn Rubb Ind.* The latex bottom fraction from centrifuged latex is washed by re-suspension in 0.4M mannitol and recovered by centrifugation. The washed bottom fraction is then subjected to repeated freezing and thawing to rupture the lutoids that are its main constituents. The lutoidic fluid, the B-serum, is recovered as the supernatant after re-centrifugation.

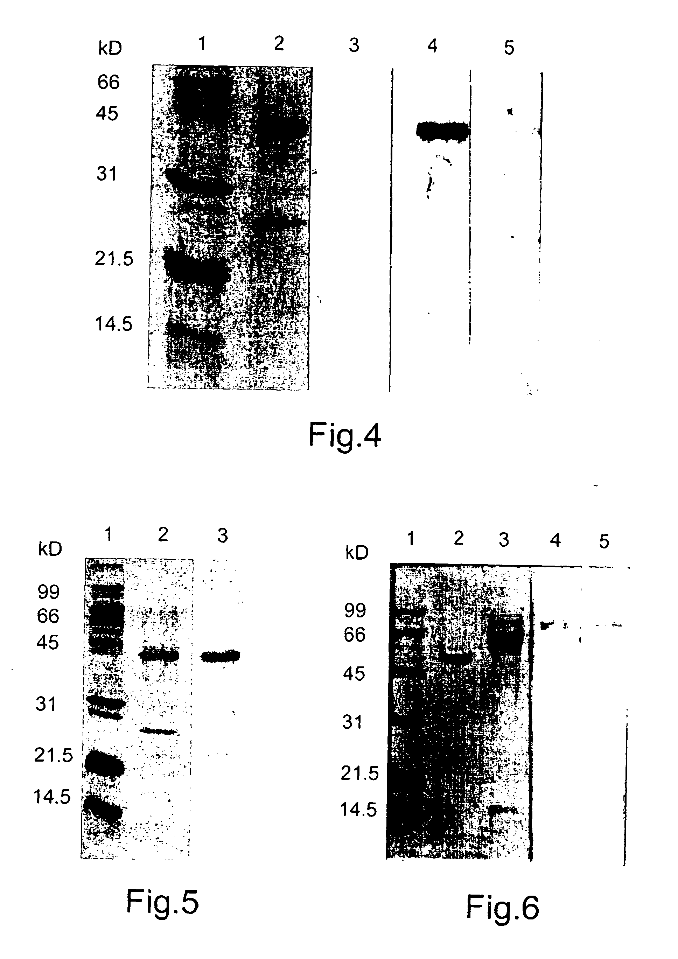

B-serum is dialysed overnight against 0.3mM sodium borate and 0.016 M boric acid pH 7 in the cold room. This is followed with filtration through Whatman No. 1 filter paper. Ten ml of the filtered B-serum is loaded onto carboxymethyl cellulose CM32 (Whatman) column (20 cm x 1.5 cm) equilibrated with 0.09 M sodium borate and 0.016 M boric acid, pH 8.6. Proteins are eluted with a gradient of 150 ml of 0.09M sodium borate and 0.016 M boric acid, pH 8.6 against 150 ml of 0.9M sodium borate and 0.16 M boric acid, pH 8.6. Two ml fractions are collected at a rate of 2.6 min/fraction. The unretarded materials (i.e. fractions 3 to 11) are loaded into a DE 52 (Whatman) column (12 cm x 1.5 cm) equlibrated with 0.1 M Tris-HCl, pH 8. The protein is eluted with a gradient of 0 - 0.5 M NaCl in the same buffer. Fractions containing proteins of about 43 kDa, as determined by SDS-polyacrylamide gel electrophoresis, are tested for immunoglobulin IgE binding with serum latex-allergic patients. The fractions containing ALP are identified and pooled. The approximate molecular weight of ALP determined by comparing the migration of ALP with that of various calibration markers is 42 kDa (Figure 4 lane 2).

### Example 2. Molecular weight and isoelectric point determination and amino acid sequencing

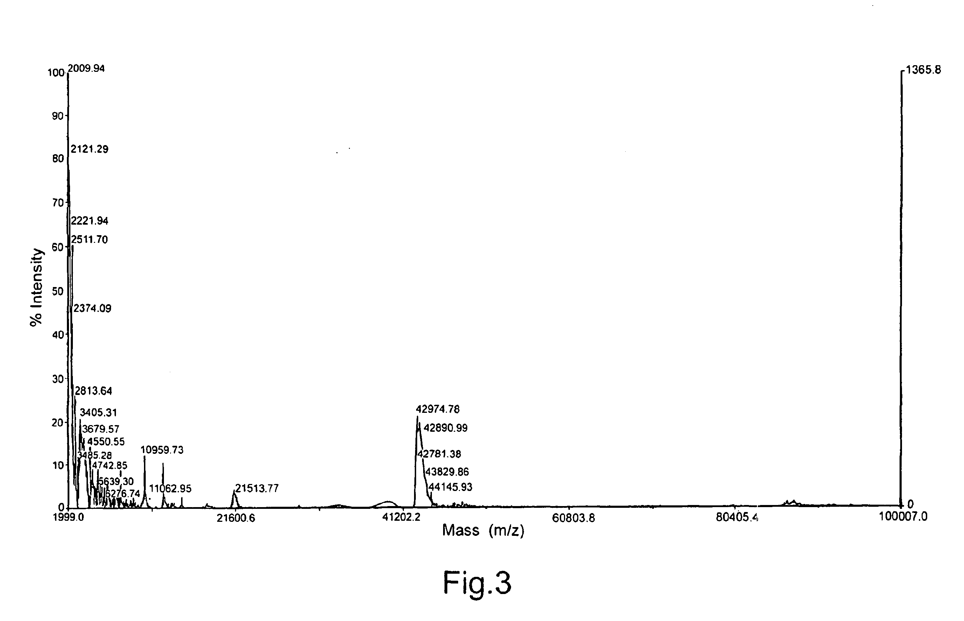

The accurate molecular weight of the allergenic latex protein, ALP, determined by mass spectrometry is 42975. The matrix assisted laser desorption ionisation mass spectrometry (MALDI-MS) spectrum of the protein sample is shown in Figure 3.

The isolectric point (pI) of ALP is determined by isoelectric focusing (IEF). The migration of the protein on the IEF gel is measured and compared with protein calibration standards of known pI. The pI of ALP is estimated to be 4.7.

The protein is found to be blocked at the N-terminal. *In situ* digestion by trypsin resulted in several fragments. Partial amino acid sequences are obtained for three of these fragments.
Sequence 1 (SEQ ID NO. 1): YLDVQYSQFR
Sequence 2 (SEQ ID NO. 2): YSLFSEPEK
Sequence 3 (SEQ ID NO. 3): LPTTIIPAHGGFSSR
where the letters of the alphabet are accepted abbreviations for individual amino acids.

Sequence 1 is derived from a peptide of 1319 Da. The amino acid sequence data obtained by mass spectrometry is compared against the protein sequence database of the National Centre for Biotechnology Information (NCBI), USA, using the BLAST algorithm. The search revealed that the amino acid sequences had partial homology with the "early nodule-specific protein" of *Glycine max.*

Sequence 2 is derived from a peptide of 1100 Da. The sequence showed partial homology with "early nodule-specific protein" of *Medicago truncatula.*

Sequence 3 is derived from a peptide of 1556 Da. The sequence showed partial homology with "early nodule-specific protein" of *Glycine max.*

### Example 3. Determination of glycosylation of ALP

To demonstrate that a carbohydrate is bound to the ALP protein (rendering ALP a 'glycosylated protein' or glycoprotein), purified proteins are separated by SDS-polyacrylamide gel electrophoresis (SDS-PAGE) on 15% gels and transferred electrophoretically to a nitrocellulose membrane to obtain a Western Blot. The membrane is washed with phosphate buffered saline (PBS) and then immersed into 10 mM sodium periodate/EDTA with agitation in the dark for 20 min. Following this, the membrane is washed three times with PBS for 10 minutes each cycle. The membrane is next transferred to a solution made up of biotin-hydrazide in sodium acetate/EDTA and agitation is carried out for 60 min. After washing three cycles with Tris-buffered saline (TBS), the membrane is blocked and then washed over another three cycles with TBS. The membrane is then immersed in strepavidin-alkaline phosphatase for 60 min. After another three cycles of washing with TBS, the membrane is immersed in a solution of 5-bromo-4-chloro-3-indolyl phosphate/nitro blue tetrazolium (BCIP/NBT) substrate. The appearance of the coloured alkaline phosphatase reaction product indicated the presence of the carbohydrate component of the glycoprotein (Figure 5).

### Example 4. Production of antibodies against ALP

Both polyclonal antibodies and monoclonal antibodies are successfully developed against ALP.

### Polyclonal antibodies against ALP

A pure preparation of ALP (approximately 0.5 ml of 0.5 mg ALP/ml) in phosphate buffered saline (PBS) is mixed with an equal volume of complete Freunds' adjuvant and this antigen mixture is injected subcutaneously into the back of rabbits. Seven booster dose of the same antigen formulation, but with incomplete Freunds' adjuvant, are administered at two week intervals. Blood is drawn from the rabbits to obtain the anti-serum that contained polyclonal antibodies against ALP.

To demonstrate polyclonal antibody binding to ALP, a Western blot of the protein on to nitrocellulose membrane is prepared as in Example 3. The nitrocellulose membrane is blocked with 5% non-fat milk in PBS and then incubated for 90 min with anti-ALP polyclonal antibodies (diluted 1:800 in PBS-milk) as the primary antibody. After three cycles of washing with PBS-milk, the nitrocellulose membrane is incubated for 1 h with the secondary antibody, anti-rabbit IgG conjugated to alkaline phosphatase. After a further three cycles of washing with PBS-milk, the nitrocellulose membrane is incubated for 10 min in Tris buffered saline (TBS) before being immersed in 5-bromo-4-chloro-3-indolyl phosphate/nitro blue tetrazolium (BCIP/NBT) substrate to generate the coloured alkaline phosphatase reaction product. The binding of polyclonal antibodies to ALP on a Western blot of the protein after separation of the protein by SDS-polyacrylamide gel electrophoresis is shown in Figure 5 (lane 4).

### Monoclonal antibodies against ALP

Spleen cells from a Balb/c mouse immunized with latex C-serum are fused with mouse myeloma cells following protocols previously described by Kohler and Milstein (12,13). The resulting hybridoma cells are screened for antibodies specific to C-serum proteins. Selected hybridomas are recloned twice and monoclonal antibodies secreted are used either in unpurified form in hybridoma cell supernatants or as preparations purified by affinity chromatography.

To demonstrate monoclonal antibody binding to ALP, a Western blot of the protein on to nitrocellulose membrane and processed as for the polyclonal antibody, except that monoclonal antibody against ALP is used as the primary antibody, while anti-mouse IgG conjugated to alkaline phosphatase is used as the secondary antibody. The binding of monoclonal antibodies to ALP on a Western blot of the protein after separation of the protein by SDS-polyacrylamide gel electrophoresis is shown in Figure 4 (lane 5).

### Recombinant monoclonal antibodies against ALP

An anticipated variation of the conventional monoclonal antibody is the recombinant antibody whereby the antibody can be generated using a specific segment of DNA that encodes the amino acid sequence of the antibody or a functional fragment of the antibody such as a single chain variable fragment. There are several approaches to the development of recombinant antibodies, of which an example is outlined here. Antibody gene libraries are first constructed, for example, by PCR-amplification from B-lymphocyte cDNA. To screen these libraries, antibodies are displayed on the surface of microorganisms containing the antibody's gene (phage display). They are challenged with the antigen protein to identify specific clones producing an antibody that bind to this protein. Once the organism bearing the antibody gene is identified, specific clones can then be amplified and used to produce the antibody fragment in *E*. *coli* or other suitable organism.

### Example 5. Demonstration of protein allergenicity

A Western blot of the purified protein is incubated with blood serum from latex allergic patients to determine if IgE (the immunoglobulin that mediates the allergic reaction) bound to the protein. Binding of IgE to the protein indicated that the protein is allergenic.

To detect protein-IgE binding in Western blots, a similar procedure as in Example 2 is followed, except that the nitrocellulose membrane is incubated overnight with serum pooled from several latex-allergic patients (diluted 1:5.25 in PBS-milk and 0.05% sodium azide) as the primary antibody. Anti-human IgE conjugated to alkaline phosphatase served as the secondary antibody. The binding of human IgE to ALP on a Western blot of the protein after separation of the protein by SDS-polyacrylamide gel electrophoresis is shown in Figure 4 (lane 3).

### Example 6. Preparation and cloning of the cDNA encoding ALP

The complementary DNA (cDNA) encoding the amino acid sequence of ALP can be cloned and multiplied in a host such as a micro-organism. The micro-organism can be selected from the group consisting of bacteria, yeast, and viruses. Higher plant cells can also be used as vectors. The ALP protein, in recombinant form, can then be synthesised in the same host or in an alternative host. The following is a description of the method used to clone the cDNA of ALP. Standard methods are used in the preparation and purification of DNA, mini- and maxipreps, DNA purification, restriction endonuclease digestions, agarose gel electrophoresis, ligations, transformations and poly(A)⁺ mRNA isolation by oligo(dT) cellulose column chromatography.

### Preparation of latex mRNA

Latex is collected by tapping the *Hevea brasiliensis* tree. Before the tree is tapped, it is fitted with a sterilised drainage spout. Immediately upon tapping, the incision and spout are washed with about 20 ml of 2x RNA extraction buffer (0.1 mol Tris-HCl, 0.3 mol LiCl, 0.01 mol EDTA, 10% SDS, pH 9.5). The latex is then washed down with 100 ml of RNA extraction buffer to a total collected volume of 200 ml in a sterile conical flask. In the laboratory, the latex is mixed well and centrifuged in polyallomer tubes at 112,700 g for 30 minutes at 15° C. The aqueous phase is gently decanted into sterile centrifuge tubes and subsequent processing of the aqueous phase to isolate total RNA is performed according to the method of Prescott and Martin (1987) *Plant Mol Biol Rep.*

### Synthesis of ALP cDNA

First strand cDNA synthesis is prepared by reverse transcribing 1 microgram of total latex RNA in 20 µl volume using the GeneRacer^{™} Kit (Invitrogen , USA) as per the vendor's instructions. Synthesis of cDNA is accomplished by PCR amplification.

The cDNA is amplified by PCR without prior purification. Each reaction is performed in a 50 µl volume containing 2 µl of the first strand reaction above, 12.5 µM of GeneRacer^{™} 3' Primer ( 5'-GCTGTCAACGATACGCTACGTAACG-3' (SEQ ID NO. 4) where A,G,C and T are the abbreviations for the nucleotides bases adenine, guanine, cytosine and thymine respectively), 12.5 µM of specific primer, 0.2 mMol dATP, 0.2 mMol dTTP, 0.2 mMol dCTP, 0.2 mMol dGTP, pH 7.5 , 1 unit of Taq High Fidelity (Roche Diagnostics GmbH), 10 mM Tris-Hcl, 1.5 mM MgCl₂, 50 mM KCl, pH 8.3, and overlayed with 50 µl of mineral oil. The PCR reaction took place in a thermocycler following the manufacturer's instructions. A second round of PCR is performed as previously but using 5 µl of the first round PCR as the template. 20 µl of the PCR amplification product is used for analysis on a 1.0 % agarose gel stained with ethidium bromide.

### Cloning of ALP cDNA.

The PCR product (about 1.5 Kb, in size) is ligated into the TOPO^{®} vector (Invitrogen , USA) as per the vendor's instructions. The ligate (2 µl) is used for the transformation of One Shot^{®} TOP10 Chemically Competent *Escherichia coli* (Invitrogen , USA) to ampicillin resistance. After incubating overnight at 37°C in agar medium containing ampicillin (100 µg/ml), transformants were picked by Xgal (80 µg/ml)/IPTG (3 mmol/L) colour selection. The picked clones are screened by miniprep assay using the Wizard^{®} SV Minipreps DNA Purification System (Promega , USA) as per the vendor's instructions. 1 ug of the selected clones are then sent for nucleic acid sequencing.

The DNA sequences (1394 basepairs, Figure 1 and SEQ ID NO. 11) are translated into the amino acids that they encoded (Figure 2 and SEQ ID NO. 12). The amino acid sequence encompassed the following segments:
1) ctaccaactactattatacctgctcatggtggatttagt (SEQ ID NO. 5: at position 384 to 422) encodes the peptide LPTTIIPAHGGFS (SEQ ID NO. 6).
2) taccttgatgtccaatattcgcaattccgg (SEQ ID NO. 7: at position 429 to 458) encodes the peptide YLDVQYSQFR (SEQ ID NO. 8)
3) tattctttattcagtgagccagaaaaa (SEQ ID NO. 9: at position 897 to 923) encodes the peptide YSLFSEPEK (SEQ ID NO. 10)
where a,g,c and t are the abbreviations for the nucleotides bases adenine, guanine, cytosine and thymine respectively. As noted earlier, these three protein domains had been independently identified by mass spectrometry. The applicants note that minor variations of the DNA sequence would not alter substantially the basic characteristics of the peptide that the DNA encodes.

### Example 7. Over-expression of recombinant ALP

There are several commercial kits that can be used for the over-expression of the allergenic latex protein. In this example, the pMAL-c2 protein fusion and purification system (New England Biolabs, USA) is used to overexpress recombinant protein from its cloned cDNA. The procedures used for the induction of fusion protein overproduction, affinity chromatography purification, cleavage of fusion protein by factor Xa protease, and purification of the target protein by hydroxyapatite chromatography are according to the vendor's instructions. In this example, isopropyl thiogalactoside (IPTG) is used as an inducer for the expression system.

The ALP cDNA is subcloned into the vector pMAL-c2 in the same translational reading frame as the *malE* gene of the vector. The bacterial cells are grown overnight at 37°C on an LB indicator plate containing 100 µg/ml ampicillin, 10 µmol isopropyl thiogalactoside (IPTG) and 10 µg/ml Xgal. White colonies are picked and screened for the presence of the MBP fusion plasmid by miniprep assay. A positive clone is then taken for the overproduction of the REF protein. IPTG-induced *E. coli* cells are disrupted by a freeze (-20° C)/thaw cycle (ambient temperature) and sonicating (Vibra Cell, Sonics & Materials Inc., USA) in ice-water bath with 15 seconds pulses for 3 minutes. The release of fusion protein eluted from the amylose column is monitored by assaying for protein. Whereas the amino acid sequence of the peptide is determined by the cDNA sequence in the expression vector, the applicants note that minor changes in the cDNA sequence would not alter substantially the basic characteristics of the recombinant protein. Figure 6 shows the expressed recombinant protein and its binding to antibodies that had been developed against its native (natural) counterpart purified from natural rubber latex.

### Example 8. Use of ALP in immunoassays

Native or recombinant ALP or its molecular variant (a protein similar to ALP, but differing slightly, for example, in a few amino acids) can be used on its own, or in combination with an antibody developed against ALP or its molecular variant, in immunoassays. Molecular variants of ALP may occur naturally in latex or may exist as a result of laboratory manipulation. Such variants of ALP may differ only slightly from one another (e.g. by a few amino acids) and they have substantially similar basic functions or characteristics including allergenicity. Such immunoassays can be constructed in many different formats, but they basically rely on the immunological reaction between an antibody and its antigen. The antibody in this instance can be an antibody against ALP or its molecular variant, or human IgE. Its antigen can be native or recombinant ALP or its molecular variant, or a peptide that embodies the epitope site of ALP or its molecular variant.

The immunoassay can be used for the diagnosis of for the diagnosis of allergy to ALP or allergy to latex in general. In a different format, the immunoassay can be used for the detection of ALP in latex or latex products.

### Example 9. Use of ALP in immunotherapy

Immunotherapy is a preventive treatment for allergic reactions that is carried out by giving gradually increasing doses of the allergen to which the person is allergic. The incremental increases of the allergen cause the immune system to become less sensitive to the substance when the substance is encountered in the future. There are several treatment protocols for immunotherapy. As an example, immunotherapy with ALP can be carried out by injecting a purified sample of ALP into the skin of the arm. An injection may be given once a week for about 30 weeks, after which injections can be administered every two weeks. Eventually, injections can be given every four weeks. The duration of therapy may be three or four years, sometimes longer. In place of native ALP, immunotherapy may also be carried out with a suitable recombinant ALP or the molecular variant of ALP (a protein similar to ALP, but differing slightly, for example, in a few amino acids), or a peptide representing a portion of ALP or its molecular variant.

From the above description, one skilled in the art can easily ascertain the essential characteristics of the present invention, and without departing from the spirit and scope thereof, can make various changes and modifications of the invention to adapt it to various usage and conditions.

## Claims

1. An allergenic latex protein (ALP), **characterized in that** the protein has at least 80% sequence identity with SEQ ID NO. 12.

2. An allergenic latex protein (ALP) according to claim 1, having at least 90% identity with SEQ ID NO. 12.

3. An allergenic latex protein (ALP) according to claim 2, having at least 95% identity with SEQ ID NO. 12.

4. An allergenic latex protein (ALP) according to claim 3, having at least 98% identity with SEQ ID NO. 12.

5. An allergenic latex protein (ALP) according to claim 4, consisting of the sequence of SEQ ID NO. 12.

6. An allergenic latex protein (ALP) according to any preceding claim, capable of inducing an allergic reaction in persons sensitized to the protein.

7. An allergenic latex protein (ALP) according to any preceding claim, wherein the DNA sequence encoding the protein is SEQ ID NO. 11.

8. An allergenic latex protein (ALP) according to any preceding claim, wherein the protein has the following properties:
a. a molecular weight of about 42,000 Dalton;
b. an isoelectric point of about 4.7; and
c. binds with IgE of patients sensitised to the protein.

9. A protein according to any preceding claim, which further comprises an additional amino acid sequence which contains secretory or leader sequences, pro-sequences, sequences which aid in purification, or an additional sequence for stability during recombinant production.

10. A protein according to any preceding claim, which is a fusion protein.

11. A process for obtaining a protein according to any preceding claim, wherein the process comprises the following steps:
a) centrifuging the latex for obtaining the bottom fraction;
b) freeze-thawing the bottom fraction for obtaining the latex B-serum; and
c) isolating and purifying the protein from the B-serum obtained in step (b).

12. A process according to claim 11, wherein the isolation and purification of the protein are carried out via a series of chromatographic separations.

13. A process according to claim 12, wherein the chromatographic separation is ion exchange chromatography and gel filtration.

14. A DNA sequence encoding the protein according to any of claims 1-10, wherein the DNA sequence is that of SEQ ID NO. 11 or a variant having at least 95% sequence identity therewith.

15. A method for the production of a protein, according to any of claims 1 - 10, in recombinant form, comprising the steps of:
a. inserting the DNA encoding the protein into an appropriate viral vector or host cell; and
b. inducing the viral vector or host cell to express a recombinant protein.

16. A method for the production of a protein in recombinant form according to claim 15, wherein the DNA encoding the protein is SEQ ID NO. 11.

17. A method for the production of a protein in recombinant form according to claim 15 or 16, wherein the host cell is a micro-organism, a plant or an animal.

18. A method for the production of a protein in recombinant form according to claim 17, wherein the micro-organism is a bacterium or a yeast.

19. A method for the production of a protein in recombinant form according to claim 18, wherein the micro-organism is *Escherichia coli.*

20. A method for the production of a protein in recombinant form according to claim 15, wherein the inducer is preferably isopropyl thiogalactoside (IPTG) or any other suitable inducer.

21. A native protein according to any of claims 1 -10 for use in immunoassay and immunotherapy.

## Patentansprüche

1. Allergenes Latexprotein (ALP), **dadurch gekennzeichnet, daß** das Protein mindestens 80% Sequenzidentität mit SEQ ID NO. 12 hat.

2. Allergenes Latexprotein (ALP) nach Anspruch 1 mit mindestens 90% Identität mit SEQ ID NO. 12.

3. Allergenes Latexprotein (ALP) nach Anspruch 2 mit mindestens 95% Identität mit SEQ ID NO. 12.

4. Allergenes Latexprotein (ALP) nach Anspruch 3 mit mindestens 98% Identität mit SEQ ID NO. 12.

5. Allergenes Latexprotein (ALP) nach Anspruch 4, bestehend aus der Sequenz von SEQ ID NO. 12.

6. Allergenes Latexprotein (ALP) nach einem vorhergehenden Anspruch, fähig, eine allergische Reaktion bei Personen, sensibilisiert gegen das Protein, zu induzieren.

7. Allergenes Latexprotein (ALP) nach einem vorhergehenden Anspruch, wobei die DNA-Sequenz, codierend das Protein, SEQ ID NO. 11 ist.

8. Allergenes Latexprotein (ALP) nach einem vorhergehenden Anspruch, wobei das Protein die folgenden Eigenschaften hat:
a. ein Molekulargewicht von etwa 42000 Dalton;
b. einen isoelektrischen Punkt von etwa 4,7; und
c. mit IgE von Patienten, sensibilisiert gegen das Protein, bindet.

9. Protein nach einem vorhergehenden Anspruch, welches weiterhin eine zusätzliche Aminosäuresequenz umfaßt, welche sekretorische oder Leadersequenzen, Prosequenzen, Sequenzen, welche die Reinigung unterstützen, oder eine zusätzliche Sequenz für Stabilität während rekombinanter Produktion enthält.

10. Protein nach einem vorhergehenden Anspruch, welches ein Fusionsprotein ist.

11. Verfahren zum Erhalt eines Proteins nach einem vorhergehenden Anspruch, wobei das Verfahren die folgenden Schritte umfaßt:
a) Zentrifugieren des Latex zum Erhalt der Bodenfraktion;
b) Gefrier-Auftauen der Bodenfraktion zum Erhalt des Latex-B-Serums; und
c) Isolieren und Reinigen des Proteins aus dem B-Serum, erhalten in Schritt (b).

12. Verfahren nach Anspruch 11, wobei die Isolierung und Reinigung des Proteins über eine Reihe von chromatographischen Trennungen ausgeführt wird.

13. Verfahren nach Anspruch 12, wobei die chromatographische Trennung Ionenaustauschchromatographie und Gelfiltration ist.

14. DNA-Sequenz, codierend das Protein nach einem der Ansprüche 1-10, wobei die DNA-Sequenz die von SEQ ID NO. 11 oder eine Variante mit mindestens 95% Sequenzidentität damit ist.

15. Verfahren für die Herstellung eines Proteins nach einem der Ansprüche 1-10 in rekombinanter Form, umfassend die Schritte:
a. Insertieren der DNA, codierend das Protein, in einen geeigneten viralen Vektor oder in eine Wirtszelle; und
b. Induzieren des viralen Vektors oder der Wirtszelle, um ein rekombinantes Protein zu exprimieren.

16. Verfahren für die Herstellung eines Proteins in rekombinanter Form nach Anspruch 15, wobei die DNA, codierend das Protein, SEQ ID NO. 11 ist.

17. Verfahren für die Herstellung eines Proteins in rekombinanter Form nach Anspruch 15 oder 16, wobei die Wirtszelle ein Mikroorganismus, eine Pflanze oder ein Tier ist.

18. Verfahren für die Herstellung eines Proteins in rekombinanter Form nach Anspruch 17, wobei der Mikroorganismus ein Bakterium oder eine Hefe ist.

19. Verfahren für die Herstellung eines Proteins in rekombinanter Form nach Anspruch 18, wobei der Mikroorganismus *Escherichia coli* ist.

20. Verfahren für die Herstellung eines Proteins in rekombinanter Form nach Anspruch 15, wobei der Induktor vorzugsweise Isopropylthiogalactosid (IPTG) oder ein anderer geeigneter Induktor ist.

21. Natives Protein nach einem der Ansprüche 1-10 zur Verwendung in Immunoassay und Immunotherapie.

## Revendications

1. Protéine allergisante du latex (PAL), **caractérisée en ce que** la protéine a au moins 80% d'identité de séquence avec SEQ ID NO: 12.

2. Protéine allergisante du latex (PAL) selon la revendication 1, ayant au moins 90% d'identité avec SEQ ID NO: 12.

3. Protéine allergisante du latex (PAL) selon la revendication 2, ayant au moins 95% d'identité avec SEQ ID NO: 12.

4. Protéine allergisante du latex (PAL) selon la revendication 3, ayant au moins 98% d'identité avec SEQ ID NO: 12.

5. Protéine allergisante du latex (PAL) selon la revendication 4, consistant en la séquence de SEQ ID NO: 12.

6. Protéine allergisante du latex (PAL) selon l'une quelconque des revendications précédentes, capable d'induire une réaction allergique chez des personnes sensibilisées à la protéine.

7. Protéine allergisante du latex (PAL) selon l'une quelconque des revendications précédentes, dans laquelle la séquence d'ADN codant pour la protéine est SEQ ID NO: 11.

8. Protéine allergisante du latex (PAL) selon l'une quelconque des revendications précédentes, dans laquelle la protéine a les propriétés suivantes:
a. un poids moléculaire d'environ 42 000 Dalton;
b. un point isoélectrique d'environ 4,7;
c. qui se lie avec des IgE de patients sensibilisés à la protéine.

9. Protéine selon l'une quelconque des revendications précédentes, qui comprend en outre une séquence d'acides aminés supplémentaire qui contient des séquences de tête ou de sécrétion, des pro-séquences, des séquences qui aident à la purification, ou une séquence supplémentaire pour la stabilité lors d'une production recombinante.

10. Protéine selon l'une quelconque des revendications précédentes, qui est une protéine de fusion.

11. Procédé d'obtention d'une protéine selon l'une quelconque des revendications précédentes, dans lequel le procédé comprend les étapes suivantes:
a) centrifugation du latex pour obtenir la fraction inférieure;
b) congélation-décongélation de la fraction inférieure pour obtenir le sérum B du latex; et
c) isolement et purification de la protéine à partir du sérum B obtenu dans l'étape (b).

12. Procédé selon la revendication 11, dans lequel l'isolement et la purification de la protéine sont réalisés par une série de séparations par chromatographie.

13. Procédé selon la revendication 12, dans lequel la séparation par chromatographie est une chromatographie par échange d'ions et une filtration sur gel.

14. Séquence d'ADN codant pour la protéine selon l'une quelconque des revendications 1 à 10, dans laquelle la séquence d'ADN est celle de SEQ ID NO: 11 ou un variant ayant au moins 95% d'identité de séquence avec celle-ci.

15. Procédé pour la production d'une protéine, selon l'une quelconque des revendications 1 à 10, sous forme recombinante, comprenant les étapes:
a. d'insertion de l'ADN codant pour la protéine dans un vecteur viral ou une cellule hôte approprié; et
b. d'induction du vecteur viral ou de la cellule hôte pour exprimer une protéine recombinante.

16. Procédé pour la production d'une protéine sous forme recombinante selon la revendication 15, dans lequel l'ADN codant pour la protéine est de SEQ ID NO: 11.

17. Procédé pour la production d'une protéine sous forme recombinante selon la revendication 15 ou 16, dans lequel la cellule hôte est un micro-organisme, une plante ou un animal.

18. Procédé pour la production d'une protéine sous forme recombinante selon la revendication 17, dans lequel le micro-organisme est une bactérie ou une levure.

19. Procédé pour la production d'une protéine sous forme recombinante selon la revendication 18, dans lequel le micro-organisme est *Escherichia coli.*

20. Procédé pour la production d'une protéine sous forme recombinante selon la revendication 15, dans lequel l'inducteur est de préférence de l'isopropyl thiogalactoside (IPTG) ou un quelconque autre inducteur adapté.

21. Protéine native selon l'une quelconque des revendications 1 à 10 pour une utilisation dans un immunoessai ou en immunothérapie.


### SEQUENCE LISTING

<110> Malaysian Rubber Board
<120> A Protein
<130> EPP89347
<150> Malaysian Patent Application No. PI200307
   <151> 2003-02-28
<160> 12
<170> Patent In version 3.2
<210> 1
   <211> 10
   <212> PRT
   <213> Hevea brasiliensis
<400> 1
   Tyr Leu Asp Val Gln Tyr Ser Gln Phe Arg
   1               5                   10
<210> 2
   <211> 9
   <212> PRT
   <213> Hevea brasiliensis
<400> 2
   Tyr Ser Leu Phe Ser Glu Pro Glu Lys
   1               5
<210> 3
   <211> 15
   <212> PRT
   <213> Hevea brasiliensis
<400> 3
   Leu Pro Thr Thr Ile Ile Pro Ala His Gly Gly Phe Ser Ser Arg
   1               5                   10                  15
<210> 4
   <211> 25
   <212> DNA
   <213> Hevea brasiliensis
<400> 4
   gctgtcaacg atacgctacg taacg 25
<210> 5
   <211> 39
   <212> DNA
   <213> Hevea brasiliensis
<400> 5
   ctaccaacta ctattatacc tgctcatggt ggatttagt 39
<210> 6
   <211> 13
   <212> PRT
   <213> Hevea brasiliensis
<400> 6
   Leu Pro Thr Thr Ile Ile Pro Ala His Gly Gly Phe Ser
   1 5 10
<210> 7
   <211> 30
   <212> DNA
   <213> Hevea brasiliensis
<400> 7
   taccttgatg tccaatattc gcaattccgg 30
<210> 8
   <211> 10
   <212> PRT
   <213> Hevea brasiliensis
<400> 8
   Tyr Leu Asp Val Gln Tyr Ser Gln Phe Arg
   1 5 10
<210> 9
   <211> 27
   <212> DNA
   <213> Hevea brasiliensis
<400> 9
   cattctttat tcagtgagcc agaaaaa 27
<210> 10
   <211> 9
   <212> PRT
   <213> Hevea brasiliensis
<400> 10
   Tyr Ser Leu Phe Ser Glu Pro Glu Lys
   1 5
<210> 11
   <211> 1394
   <212> DNA
   <213> Hevea brasiliensis
<400> 11
   acgcgggggc gttaacactt ggtttttgct tccacttcat ggagttccct gaaaccaata      60
   acaaccctat catcactctc tctttcttat tatgcatgct ttccctagct tatgcttccg     120
   aaacctgtga ttttccagca atctttaact tcggcgactc caattccgat accggtggca     180
   aggcagctgc cttttatcct cttaaccctc cttatggaga gactttcttt cacaggtcga     240
   caggaaggta ctctgatgga aggctcataa tagattttat cgccgagagt ttcaatctcc     300
   catatctgag tccatatctt agttccctgg gaagcaactt caaacatggt gcagattttg     360
   ccacagcagg atccaccatt aaactaccaa ctactattat acctgctcat ggtggattta     420
   gtccattcta ccttgatgtc caatattcgc aattccggca attcataccc agatcacagt     480
   ttatcaggga aactggaggc atatttgctg aattggtgcc cgaggaatat tattttgaga     540
   aagctttata cacattcgat attggtcaaa atgatcttac agaaggattc ttgaacttaa     600
   ctgtggaaga agtgaatgca actgtccctg atcttgtgaa tagcttctca gcaaacgtta     660
   agaaaatata cgatttggga gctagaacat tttggattca caacacagga ccaattggtt     720
   gtctttcatt cattttaacg tattttccct gggcagaaaa ggatagtgca ggctgtgcaa     780
   aagcttacaa tgaagttgct cagcatttta atcacaagtt gaaggagatc gttgctcaac     840
   tcaggaagga tttgccttta gctacattcg tccacgttga catctattct gtcaagtatt     900
   ctttattcag tgagccagaa aaacacggtt tcgagtttcc acttataaca tgttgtggct     960
   acggaggaaa gtacaatttt agtgttactg ctccatgtgg agatacagtt acagcagacg    1020
   acggtaccaa aatagttgtg ggttcatgtg cttgcccttc agttcgagta aattgggatg    1080
   gagctcacta cactgaagct gccaatgaat attttttcga ccagatttct acaggagcct    1140
   tctctgatcc ccctgttcca ttgaatatgg catgtcataa aactgaatca ttgaggacat    1200
   tagcctctgt ataggttata tgaaagtgct ttgctgaaag cccgctaata aaatgaggaa    1260
   taataataaa tgagaaacca ttgattatgt taggattcac ttggtttcta tcataataat    1320
   ctatctgttg tatatacaac agttgtatga aatagtttct tgtaataaag acttgtcttt    1380
   ctccggtttc ccta                                                      1394
<210> 12
   <211> 464
   <212> PRT
   <213> Hevea brasiliensis
<220>
   <221> misc_feature
   <222> (404)..(404)
   <223> Xaa can be any naturally occurring amino acid
<220>
   <221> misc_feature
   <222> (407)..(407)
   <223> Xaa can be any naturally occurring amino acid
<220>
   <221> misc_feature
   <222> (418)..(418)
   <223> Xaa can be any naturally occurring amino acid
<220>
   <221> misc_feature
   <222> (438)..(439)
   <223> Xaa can be any naturally occurring amino acid
<220>
   <221> misc_feature
   <222> (451)..(451)
   <223> Xaa can be any naturally occurring amino acid
<400> 12
   Ala Gly Ala Leu Thr Leu Gly Phe Cys Phe His Phe Met Glu Phe Pro
   1               5                   10                  15
   Glu Thr Asn Asn Asn Pro Ile Ile Thr Leu Ser Phe Leu Leu Cys Met
               20                  25                  30
   Leu Ser Leu Ala Tyr Ala Ser Glu Thr Cys Asp Phe Pro Ala Ile Phe
           35                  40                  45
   Asn Phe Gly Asp Ser Asn Ser Asp Thr Gly Gly Lys Ala Ala Ala Phe
       50                  55                  60
   Tyr Pro Leu Asn Pro Pro Tyr Gly Glu Thr Phe Phe His Arg Ser Thr
   65                  70                  75                  80
   Gly Arg Tyr Ser Asp Gly Arg Leu Ile Ile Asp Phe Ile Ala Glu Ser
                   85                  90                  95
   Phe Asn Leu Pro Tyr Leu Ser Pro Tyr Leu Ser Ser Leu Gly Ser Asn
               100                 105                 110
   Phe Lys His Gly Ala Asp Phe Ala Thr Ala Gly Ser Thr Ile Lys Leu
           115                 120                 125
   Pro Thr Thr Ile Ile Pro Ala His Gly Gly Phe Ser Pro Phe Tyr Leu
       130                 135                 140
   Asp Val Gln Tyr Ser Gln Phe Arg Gln Phe Ile Pro Arg Ser Gln Phe
   145                 150                 155                 160
   Ile Arg Glu Thr Gly Gly Ile Phe Ala Glu Leu Val Pro Glu Glu Tyr
                   165                 170                 175
   Tyr Phe Glu Lys Ala Leu Tyr Thr Phe Asp Ile Gly Gln Asn Asp Leu
               180                 185                 190
   Thr Glu Gly Phe Leu Asn Leu Thr Val Glu Glu Val Asn Ala Thr Val
           195                 200                 205
   Pro Asp Leu Val Asn Ser Phe Ser Ala Asn Val Lys Lys Ile Tyr Asp
       210                 215                 220
   Leu Gly Ala Arg Thr Phe Trp Ile His Asn Thr Gly Pro Ile Gly Cys
   225                 230                 235                 240
   Leu Ser Phe Ile Leu Thr Tyr Phe Pro Trp Ala Glu Lys Asp Ser Ala
                   245                 250                 255
   Gly Cys Ala Lys Ala Tyr Asn Glu Val Ala Gln His Phe Asn His Lys
               260                 265                 270
   Leu Lys Glu Ile Val Ala Gln Leu Arg Lys Asp Leu Pro Leu Ala Thr
           275                 280                 285
   Phe Val His Val Asp Ile Tyr Ser Val Lys Tyr Ser Leu Phe Ser Glu
       290                 295                 300
   Pro Glu Lys His Gly Phe Glu Phe Pro Leu Ile Thr Cys Cys Gly Tyr
   305                 310                 315                 320
   Gly Gly Lys Tyr Asn Phe Ser Val Thr Ala Pro Cys Gly Asp Thr Val
                   325                 330                 335
   Thr Ala Asp Asp Gly Thr Lys Ile Val Val Gly Ser Cys Ala Cys Pro
               340                 345                 350
   Ser Val Arg Val Asn Trp Asp Gly Ala His Tyr Thr Glu Ala Ala Asn
           355                 360                 365
   Glu Tyr Phe Phe Asp Gln Ile Ser Thr Gly Ala Phe Ser Asp Pro Pro
       370                 375                 380
   Val Pro Leu Asn Met Ala Cys His Lys Thr Glu Ser Leu Arg Thr Leu
   385                 390                 395                 400
   Ala Ser Val Xaa Val Ile Xaa Lys Cys Phe Ala Glu Ser Pro Leu Ile
                   405                 410                 415
   Lys Xaa Gly Ile Ile Ile Asn Glu Lys Pro Leu Ile Met Leu Gly Phe
               420                 425                 430
   Thr Trp Phe Leu Ser Xaa Xaa Ser Ile Cys Cys Ile Tyr Asn Ser Cys
           435                 440                 445
   Met Lys Xaa Phe Leu Val Ile Lys Thr Cys Leu Ser Pro Val Ser Leu
       450                 455                 460

### SEQUENCE LISTING

<110> Malaysian Rubber Board
<120> A Protein
<130> EPP89347
<150> Malaysian Patent Application No. PI200307
   <151> 2003-02-28
<160> 12
<170> Patent In version 3.2
<210> 1
   <211> 10
   <212> PRT
   <213> Hevea brasiliensis
<400> 1
   Tyr Leu Asp Val Gln Tyr Ser Gln Phe Arg
   1               5                   10
<210> 2
   <211> 9
   <212> PRT
   <213> Hevea brasiliensis
<400> 2
   Tyr Ser Leu Phe Ser Glu Pro Glu Lys
   1               5
<210> 3
   <211> 15
   <212> PRT
   <213> Hevea brasiliensis
<400> 3
   Leu Pro Thr Thr Ile Ile Pro Ala His Gly Gly Phe Ser Ser Arg
   1               5                   10                  15
<210> 4
   <211> 25
   <212> DNA
   <213> Hevea brasiliensis
<400> 4
   gctgtcaacg atacgctacg taacg 25
<210> 5
   <211> 39
   <212> DNA
   <213> Hevea brasiliensis
<400> 5
   ctaccaacta ctattatacc tgctcatggt ggatttagt 39
<210> 6
   <211> 13
   <212> PRT
   <213> Hevea brasiliensis
<400> 6
   Leu Pro Thr Thr Ile Ile Pro Ala His Gly Gly Phe Ser
   1               5                   10
<210> 7
   <211> 30
   <212> DNA
   <213> Hevea brasiliensis
<400> 7
   taccttgatg tccaatattc gcaattccgg 30
<210> 8
   <211> 10
   <212> PRT
   <213> Hevea brasiliensis
<400> 8
   Tyr Leu Asp Val Gln Tyr Ser Gln Phe Arg
   1               5                   10
<210> 9
   <211> 27
   <212> DNA
   <213> Hevea brasiliensis
<400> 9
   tattctttat tcagtgagcc agaaaaa 27
<210> 10
   <211> 9
   <212> PRT
   <213> Hevea brasiliensis
<400> 10
   Tyr Ser Leu Phe Ser Glu Pro Glu Lys
   1               5
<210> 11
   <211> 1394
   <212> DNA
   <213> Hevea brasiliensis
<400> 11
   acgcgggggc gttaacactt ggtttttgct tccacttcat ggagttccct gaaaccaata 60
   acaaccctat catcactctc tctttcttat tatgcatgct ttccctagct tatgcttccg 120
   aaacctgtga ttttccagca atctttaact tcggcgactc caattccgat accggtggca 180
   aggcagctgc cttttatcct cttaaccctc cttatggaga gactttcttt cacaggtcga 240
   caggaaggta ctctgatgga aggctcataa tagattttat cgccgagagt ttcaatctcc 300
   catatctgag tccatatctt agttccctgg gaagcaactt caaacatggt gcagattttg 360
   ccacagcagg atccaccatt aaactaccaa ctactattat acctgctcat ggtggattta 420
   gtccattcta ccttgatgtc caatattcgc aattccggca attcataccc agatcacagt 480
   ttatcaggga aactggaggc atatttgctg aattggtgcc cgaggaatat tattttgaga 540
   aagctttata cacattcgat attggtcaaa atgatcttac agaaggattc ttgaacttaa 600
   ctgtggaaga agtgaatgca actgtccctg atcttgtgaa tagcttctca gcaaacgtta 660
   agaaaatata cgatttggga gctagaacat tttggattca caacacagga ccaattggtt 720
   gtctttcatt cattttaacg tattttccct gggcagaaaa ggatagtgca ggctgtgcaa 780
   aagcttacaa tgaagttgct cagcatttta atcacaagtt gaaggagatc gttgctcaac 840
   tcaggaagga tttgccttta gctacattcg tccacgttga catctattct gtcaagtatt 900
   ctttattcag tgagccagaa aaacacggtt tcgagtttcc acttataaca tgttgtggct 960
   acggaggaaa gtacaatttt agtgttactg ctccatgtgg agatacagtt acagcagacg 1020
   acggtaccaa aatagttgtg ggttcatgtg cttgcccttc agttcgagta aattgggatg 1080
   gagctcacta cactgaagct gccaatgaat attttttcga ccagatttct acaggagcct 1140
   tctctgatcc ccctgttcca ttgaatatgg catgtcataa aactgaatca ttgaggacat 1200
   tagcctctgt ataggttata tgaaagtgct ttgctgaaag cccgctaata aaatgaggaa 1260
   taataataaa tgagaaacca ttgattatgt taggattcac ttggtttcta tcataataat 1320
   ctatctgttg tatatacaac agttgtatga aatagtttct tgtaataaag acttgtcttt 1380
   ctccggtttc ccta 1394
<210> 12
   <211> 464
   <212> PRT
   <213> Hevea brasiliensis
<220>
   <221> misc_feature
   <222> (404)..(404)
   <223> Xaa can be any naturally occurring amino acid
<220>
   <221> misc_feature
   <222> (407)..(407)
   <223> Xaa can be any naturally occurring amino acid
<220>
   <221> misc_feature
   <222> (418)..(418)
   <223> Xaa can be any naturally occurring amino acid
<220>
   <221> misc_feature
   <222> (438)..(439)
   <223> Xaa can be any naturally occurring amino acid
<220>
   <221> misc_feature
   <222> (451)..(451)
   <223> Xaa can be any naturally occurring amino acid
<400> 12
   Ala Gly Ala Leu Thr Leu Gly Phe Cys Phe His Phe Met Glu Phe Pro
   1               5                   10                  15
   Glu Thr Asn Asn Asn Pro Ile Ile Thr Leu Ser Phe Leu Leu Cys Met
               20                  25                  30
   Leu Ser Leu Ala Tyr Ala Ser Glu Thr Cys Asp Phe Pro Ala Ile Phe
           35                  40                  45
   Asn Phe Gly Asp Ser Asn Ser Asp Thr Gly Gly Lys Ala Ala Ala Phe
       50                  55                  60
   Tyr Pro Leu Asn Pro Pro Tyr Gly Glu Thr Phe Phe His Arg Ser Thr
   65                  70                  75                  80
   Gly Arg Tyr Ser Asp Gly Arg Leu Ile Ile Asp Phe Ile Ala Glu Ser
                   85                  90                  95
   Phe Asn Leu Pro Tyr Leu Ser Pro Tyr Leu Ser Ser Leu Gly Ser Asn
               100                 105                 110
   Phe Lys His Gly Ala Asp Phe Ala Thr Ala Gly Ser Thr Ile Lys Leu
           115                 120                 125
   Pro Thr Thr Ile Ile Pro Ala His Gly Gly Phe Ser Pro Phe Tyr Leu
       130                 135                 140
   Asp Val Gln Tyr Ser Gln Phe Arg Gln Phe Ile Pro Arg Ser Gln Phe
   145                 150                 155                 160
   Ile Arg Glu Thr Gly Gly Ile Phe Ala Glu Leu Val Pro Glu Glu Tyr
                   165                 170                 175
   Tyr Phe Glu Lys Ala Leu Tyr Thr Phe Asp Ile Gly Gln Asn Asp Leu
               180                 185                 190
   Thr Glu Gly Phe Leu Asn Leu Thr Val Glu Glu Val Asn Ala Thr Val
           195                 200                 205
   Pro Asp Leu Val Asn Ser Phe Ser Ala Asn Val Lys Lys Ile Tyr Asp
       210                 215                 220
   Leu Gly Ala Arg Thr Phe Trp Ile His Asn Thr Gly Pro Ile Gly Cys
   225                 230                 235                 240
   Leu Ser Phe Ile Leu Thr Tyr Phe Pro Trp Ala Glu Lys Asp Ser Ala
                   245                 250                 255
   Gly Cys Ala Lys Ala Tyr Asn Glu Val Ala Gln His Phe Asn His Lys
               260                 265                 270
   Leu Lys Glu Ile Val Ala Gln Leu Arg Lys Asp Leu Pro Leu Ala Thr
           275                 280                 285
   Phe Val His Val Asp Ile Tyr Ser Val Lys Tyr Ser Leu Phe Ser Glu
       290                 295                 300
   Pro Glu Lys His Gly Phe Glu Phe Pro Leu Ile Thr Cys Cys Gly Tyr
   305                 310                 315                 320
   Gly Gly Lys Tyr Asn Phe Ser Val Thr Ala Pro Cys Gly Asp Thr Val
                   325                 330                 335
   Thr Ala Asp Asp Gly Thr Lys Ile Val Val Gly Ser Cys Ala Cys Pro
               340                 345                 350
   Ser Val Arg Val Asn Trp Asp Gly Ala His Tyr Thr Glu Ala Ala Asn
           355                 360                 365
   Glu Tyr Phe Phe Asp Gln Ile Ser Thr Gly Ala Phe Ser Asp Pro Pro
       370                 375                 380
   Val Pro Leu Asn Met Ala Cys His Lys Thr Glu Ser Leu Arg Thr Leu
   385                 390                 395                 400
   Ala Ser Val Xaa Val Ile Xaa Lys Cys Phe Ala Glu Ser Pro Leu Ile
                   405                 410                 415
   Lys Xaa Gly Ile Ile Ile Asn Glu Lys Pro Leu Ile Met Leu Gly Phe
               420                 425                 430
   Thr Trp Phe Leu Ser Xaa Xaa Ser Ile Cys Cys Ile Tyr Asn Ser Cys
           435                 440                 445
   Met Lys Xaa Phe Leu Val Ile Lys Thr Cys Leu Ser Pro Val Ser Leu
       450                 455                 460